(19) Europäisches Patentamt
European Patent Office
Office européen des brevets

(11) EP 3 413 894 B1

(12) **EUROPEAN PATENT SPECIFICATION**

(45) Date of publication and mention of the grant of the patent:
**11.03.2020 Bulletin 2020/11**

(21) Application number: **17704548.1**

(22) Date of filing: **10.02.2017**

(51) Int Cl.:
***A61K 31/519*** *(2006.01)* ***A61P 37/00*** *(2006.01)*
***A61K 45/06*** *(2006.01)*

(86) International application number:
**PCT/IB2017/050743**

(87) International publication number:
**WO 2017/118965 (13.07.2017 Gazette 2017/28)**

(54) **USE OF INHIBITORS OF THE ACTIVITY OR FUNCTION OF PI3K FOR THE TREATMENT OF PRIMARY SJÖGREN'S SYNDROME**

VERWENDUNG VON INHIBITOREN DER AKTIVITÄT ODER FUNKTION VON PI3K ZUR BEHANDLUNG DES PRIMÄREN SJÖGREN-SYNDROMS

UTILISATION D'INHIBITEURS DE L'ACTIVITÉ OU LA FONCTION DE PI3K POUR LE TRAITEMENT DU SYNDROME DE SJÖGREN PRIMAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.02.2016 EP 16155123**
**29.08.2016 EP 16186188**

(43) Date of publication of application:
**19.12.2018 Bulletin 2018/51**

(73) Proprietor: **Novartis AG**
**4056 Basel (CH)**

(72) Inventors:
- **BIETH, Bruno**
**4002 Basel (CH)**
- **BURKHART, Christoph**
**4002 Basel (CH)**
- **CHRIST, Andreas**
**4002 Basel (CH)**
- **DE BUCK, Stefan**
**4002 Basel (CH)**
- **KALIS, Christoph**
**4002 Basel (CH)**
- **LINDGREN, Sam**
**4002 Basel (CH)**

(74) Representative: **Nederlandsch Octrooibureau**
**P.O. Box 29720**
**2502 LS The Hague (NL)**

(56) References cited:
**WO-A1-2012/004299 WO-A1-2013/001445**
**WO-A1-2015/162584**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 413 894 B1

## Description

### FIELD OF THE INVENTION

The invention relates to uses of inhibitors of the activity or function of the phosphatidylinositol 3-kinase family (hereinafter PI3K inhibitors), wherein said inhibitors have an inhibitory action on the PI3K isoform delta and/or pharmaceutically acceptable salts and/or solvates thereof for the treatment of primary Sjögren's Syndrome. The invention relates more specifically to 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof for use in the treatment of primary Sjögren's Syndrome.

### BACKGROUND OF THE INVENTION

Sjögren's syndrome is classified as either 'primary' or 'secondary'. Primary Sjogren syndrome (pSS) occurs in the absence of another underlying rheumatic disorder, whereas secondary Sjogren syndrome is associated with another underlying rheumatic disease, such as systemic lupus erythematosus (SLE), rheumatoid arthritis (RA), or scleroderma. Primary Sjögren's syndrome is a chronic autoimmune disease in which the body's immune system attacks glands that secrete fluid for example the salivary and lacrimal glands. The immune-mediated attack on the salivary and lacrimal glands leads to the development of dry mouth and dry eyes. Other symptoms or conditions of pSS include dry skin, tiredness and fatigue sometimes up to total exhaustion, muscle pain, joint pain, stiffness and swelling of joints, vasculitis, difficulty to concentrate. Currently, there is no cure known for primary Sjögren's syndrome, but treatments can help to control the symptoms. Nonsteroidal anti-inflammatory drugs may be used to treat musculoskeletal symptoms, but also corticosteroids, immunosuppressive drugs and, disease-modifying antirheumatic drugs (DMARDs) are prescribed, most of which have adverse side effects. There is a need, therefore, for additional therapeutic advances in treating primary Sjögren's Syndrome (Holdgate N. F1000Research 2016, 5(F1000 Faculty Rev):1412).

### SUMMARY OF THE INVENTION

It has been found that PI3K delta inhibitor 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof are suitable for the treatment of primary Sjögren's Syndrome.

### DESCRIPTION OF THE DRAWINGS

**Figure 1** shows the PK/PD relationship of compound A after single oral administration to healthy human subjects

### DETAILED DESCRIPTION OF THE INVENTION

It has been found that 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof are suitable for the treatment of primary Sjögren's Syndrome.

1-{(*S*)-3-[6-(6-Methoxy-5-trifluoromethyl-pyridin-3-yl)-5,6,7,8-tetrahydro-pyrido[4,3-d]pyrimidin-4-ylamino]-pyrrolidin-1-yl}-propan-1-one

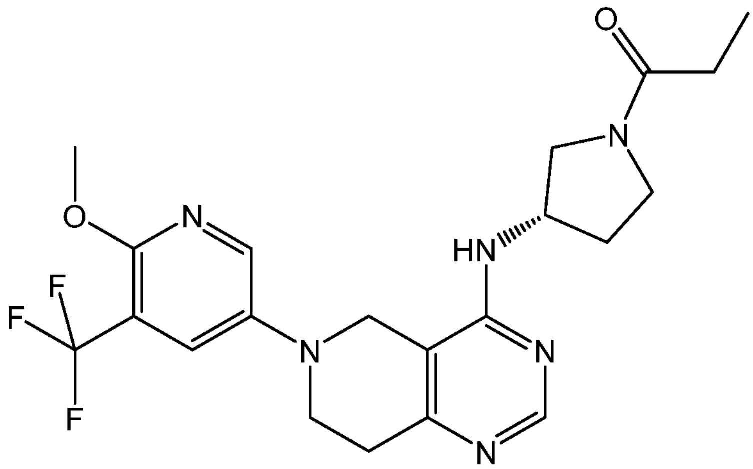

(compound **A**)

and examples of pharmaceutically acceptable salts thereof are described in Example 67 of WO2012/004299.

Phosphorylated Akt (pAkt) is a downstream effector of PI3K delta activation. 1-{(S)-3-[6-(6-Methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof is a PI3K inhibitor with a selectivity for the PI3K delta isoform (WO2012/004299). We hypothesized that Akt pathway is activated in pSS patients, and it was found that 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts are useful in the treatment of pSS.

1) Chronic B-cell hyperactivity is a consistent and prominent immunoregulatory abnormality in pSS (Hansen A et al, Arthritis Research & Therapy 2007; 9; 218). 1-{(*S*)-3-[6-(6-Methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof directly inhibits multiple B-cell functions in vitro and in vivo, for example pAkt, CD69, CD86, APC function, cytokine production and antibody production.

2) Patients with pSS have been found to express a unique profile of adhesion molecules, cytokines and chemokines including a striking overexpression of the B-cell attracting chemokine CXCL13 (BCA-1, BLC), but also of CXCL10 (IP-10) and CCL4 (MIP-1beta) which play a role in the pathogenesis of pSS (Hansen A et al, Arthritis Research & Therapy 2007; 9; 218, Lee, Y. J. et al, Rheumatology 2010; 49(9);1747-1752, Kramer JM et al, J Leukoc Biol. 2013; 94(5);1079-1089, Nishikawa A et al Arthritis Research & Therapy 2016;18;106). 1-{(S)-3-[6-(6-Methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof inhibits CXCL13 (BCA-1, BLC), but also of CXCL10 (IP-10) and CCL4 (MIP-1beta) in supernatant of BPMC derived from human helathy volunteers stimulated with CpG or anti-IgM.

3) Characteristic features in pSS include the formation of ectopic lymphoid tissue with germinal center (GC)-like structures. In healthy individuals, GCs are generated from primary B-cell follicles of secondary lymphoid organs during T-cell -dependent immune responses (Hansen A et al, Arthritis Research & Therapy 2007; 9; 218).

Histologically, the inhibition of GC formation is accompanied by an overall reduction of marginal zone B-cells (MZ B-cells) and follicular T helper cells (TFH) as measured by FACS analysis. Those cells are considered to contribute to the pathophysiology in pSS. 1-{(*S*)-3-[6-(6-Methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof reduces GC-like structures, MZ B-cells and TFH.

In one embodiment, the invention provides 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof for use in the treatment of primary Sjögren's Syndrome.

In another embodiment, the invention provides a compound for use in a method for the treatment of primary Sjögren's Syndrome, comprising administation of a therapeutically effective amount of 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof, to a subject, e.g. a human subject, in need of such treatment.

In another embodiment, the invention provides the use of 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof for the manufacture of a medicament for the treatment of primary Sjögren's Syndrome.

In another embodiment, the invention provides 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof for use in the treatment of primary Sjögren's Syndrome.

Preferred are any of the above embodiments, wherein 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one is in phosphate salt form.

In a preferred embodiment the invention relates to the phosphate salt of 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one.

As used herein, the term "subject" refers to an animal. Typically the animal is a mammal. A subject also refers to for example, primates (*e.g.*, humans, male or female), cows, sheep, goats, horses, dogs, cats, rabbits, rats, mice, fish, birds and the like. In certain embodiments, the subject is a primate. In a preferred embodiment, the subject is a human.

As used herein, the term "inhibit", "inhibition" or "inhibiting" refers to the reduction or suppression of a given condition, symptom, or disorder, or disease, or a significant decrease in the baseline activity of a biological activity or process.

As used herein, the term "treat", "treating" or "treatment" of any disease or disorder refers in one embodiment, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (*e.g.,* stabilization of a discernible symptom), physiologically, (*e.g.,* stabilization of a physical parameter), or both. In yet another embodiment, "treat", "treating" or "treatment" refers to preventing or delaying the onset or development or progression of the disease or disorder.

As used herein, a subject is "in need of' a treatment if such subject would benefit biologically, medically or in quality of life from such treatment.

As used herein, the term "administration" or "administering" of the subject compound means providing a compound of the invention and prodrugs thereof to a subject in need of treatment. Administration "in combination with" one or more further therapeutic agents includes simultaneous (concurrent) and consecutive administration in any order, and in any route of administration.

Other therapeutic agents as combination partners include for example antibodies binding to CD40, such as disclosed in WO2012/065950; inducible T cell costimulators, such as AMG557; antibodies targeting B-cell activating factor receptor (BAFF-R) such as disclosed in WO2010/007082; low dose IL-2, anti CD20 antibodies, such as rituximab; antibodies that inhibit B-cell activating factor (BAFF) such as belimumab; antibodies against the interleukin-6 receptor (IL-6R), such as tocilizumab; abatacept; or belatacept; but also cyclosporine eye drops; disease-modifying antirheumatic drugs (DMARD's), such as methotrexate, sulfasalazine, leflunomide, hydroxychloroquine and gold salts; tumor necrosis factor (TNF)-a inhibitors, such as infliximab and etanercept; non-steroidal anti-inflammatory drugs, such as ibuprofen; systemic corticosteroids, such as prednisone; or other immunosuppressive agents, such as azathioprine, mycophenolate mofetil and cyclophosphamide.

Other agents as combination partners include for example secretagogues; muscarinic receptor agonists, such as cevimeline and pilocarpine; gabapentin or pregabalin; artificial tears; artificial saliva; or vaginal estrogen cream.

Pharmaceutical composition comprising 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof and a pharmaceutically acceptable carrier. uitalbe pharmaceutically acceptable carriers are dscribed in WO2012/004299. The preferred route of administration is oral.

In one embodiment, the invention provides a combination, in particular a pharmaceutical combination, comprising a therapeutically effective amount of 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof and one or more therapeutically active agent.

In one embodiment, the invention provides a combination, in particular a pharmaceutical combination, comprising a therapeutically effective amount of the phosphate salt of 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one and one or more therapeutically active agent.

In another embodiment, the invention provides a product comprising 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts and at least one other therapeutic agent as a combined preparation for simultaneous, separate or sequential use in the treatment of pSS.

In another embodiment, the invention provides a product comprising the phosphate salt of 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one

and at least one other therapeutic agent as a combined preparation for simultaneous, separate or sequential use in the treatment of pSS.

Products provided as a combined preparation for the treatment of pSS include a composition comprising 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof and the other therapeutic agent(s) together in the same pharmaceutical composition, or the compound of Formula (I) and the other therapeutic agent(s) in separate form, e.g. in the form of a kit.

Products provided as a combined preparation for the treatment of pSS include a composition comprising the phosphate salt of 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one and the other therapeutic agent(s) together in the same pharmaceutical composition, or the compound of Formula (I) and the other therapeutic agent(s) in separate form, e.g. in the form of a kit.

Accordingly, the invention provides 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof for use in the treatment of pSS, wherein the medicament is prepared for administration with another therapeutic agent.

The invention also provides the use of another therapeutic agent for treating pSS, wherein the medicament is administered with 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof.

The invention also provides 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof for use in a method of treating pSS, wherein 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof is prepared for administration with another therapeutic agent.

The invention also provides another therapeutic agent for use in a method of treating pSS wherein the other therapeutic agent is prepared for administration with 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof.

The invention also provides 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof for use in a method of treating pSS, wherein 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof is administered with another therapeutic agent.

The invention also provides another therapeutic agent for use in a method of treating pSS, wherein the other therapeutic agent is administered with 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof.

The invention also provides 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof for use in treating pSS, wherein the patient has previously (*e.g.* within 24 hours) been treated with another therapeutic agent.

The invention also provides another therapeutic agent for use in treating pSS, wherein the patient has previously (*e.g.* within 24 hours) been treated with 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof.

The invention provides the phosphate salt of 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one for use in the treatment of pSS, wherein the medicament is prepared for administration with another therapeutic agent.

The invention also provides another therapeutic agent for use in treating pSS, wherein the medicament is administered with the phosphate salt of 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one.

The invention also provides the phosphate salt of 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one for use in a method of treating pSS, wherein the phosphate salt of 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one is prepared for administration with another therapeutic agent.

The invention also provides another therapeutic agent for use in a method of treating pSS wherein the other therapeutic agent is prepared for administration with the phosphate salt of 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one.

The invention also provides the phosphate salt of 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one for use in a method of treating pSS, wherein the phosphate salt of 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one is administered with another therapeutic agent.

The invention also provides another therapeutic agent for use in a method of treating pSS, wherein the other therapeutic agent is administered with the phosphate salt of 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one.

The invention also provides the phosphate salt of 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one for use in treating pSS, wherein the patient has previously (*e.g.* within 24 hours) been treated with another therapeutic agent.

The invention also provides another therapeutic agent for use in treating pSS, wherein the patient has previously (*e.g.* within 24 hours) been treated with the phosphate salt of 1-{(*S*)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one.

In one embodiment, the pharmaceutical composition or combination comprising 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof for treating pSS can be in unit dosage of about 10-100 mg of active ingredient for a human subject of about 50-70 kg.

In another embodiment, the pharmaceutical composition or combination comprising 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof for treating pSS can be in unit dosage of about 10-100 mg of active ingredient for a human subject of about 40-200 kg.

In one embodiment, the pharmaceutical composition or combination comprising the phosphate salt of 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one for treating pSS can be in unit dosage of about 10-100 mg of active ingredient for a human subject of about 50-70 kg.

In another embodiment, the pharmaceutical composition or combination comprising the phosphate salt of 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one for treating pSS can be in unit dosage of about 10-100 mg of active ingredient for a human subject of about 40-200 kg.

The therapeutically effective dosage of a compound, the pharmaceutical composition, or the combinations thereof, is dependent the body weight, age and individual condition, or the severity of the disorder or disease being treated. A physician or clinician of ordinary skill can readily determine the effective amount of each of the active ingredients necessary to prevent, treat or inhibit the progress of the disorder or disease.

In another embodiment, the pharmaceutical composition or combination comprising 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof for treating pSS can be in unit dosage of about 70 mg of active ingredient for a human subject of about 50-70 kg.

In another embodiment, the pharmaceutical composition or combination comprising 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof for treating pSS can be in unit dosage of about 70 mg of active ingredient for a human subject of about 40-200 kg.

In another embodiment, the pharmaceutical composition or combination comprising the phosphate salt of 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one for treating pSS can be in unit dosage of about 70 mg of active ingredient for a human subject of about 50-70 kg.

In another embodiment, the pharmaceutical composition or combination comprising the phosphate salt of 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one for treating pSS can be in unit dosage of about 70 mg of active ingredient for a human subject of about 40-200 kg.

In another embodiment, the pharmaceutical composition or combination comprising 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof for treating pSS is administered at about 70 mg of active ingredient for a human subject of about 50-70 kg, b.i.d..

In another embodiment, the pharmaceutical composition or combination comprising 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof for treating pSS is administered at about 70 mg of active ingredient for a human subject of about 40-200 kg, b.i.d..

In another embodiment, the pharmaceutical composition or combination comprising the phosphate salt of 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one for treating pSS is administered at about 70 mg of active ingredient for a human subject of about 50-70 kg, b.i.d..

In another embodiment, the pharmaceutical composition or combination comprising the phosphate salt of 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one for treating pSS is administered at about 70 mg of active ingredient for a human subject of about 40-200 kg, b.i.d..

EXPERIMENTAL DETAILS

Insofar as the production of the starting materials is not particularly described, the compounds are known or may be prepared analogously to methods known in the art or as described hereafter.

The following examples are illustrative of the invention without any limitation.

Abbreviations:

| Abbreviation | Description |
|---|---|
| Akt | see PKB |
| APC | Antigen-presenting cell |
| BCR | B cell receptor |
| BD | Becton Dickinson |
| b.i.d. | twice a day (*"bis in die"*) |
| BMMC | Bone marrow-derived mast cells |
| BSA | Bovine serum albumine |
| CsA | Cyclosporine A |
| DMSO | Dimethyl sulfoxide |
| $EC_{50}$ | Concentration leading to 50% effect |
| FACS | Fluorescence activated cell sorter |
| fMLP | N-Formylmethionyl-Lencyl-Phenylalanin |
| HEL | Hen egg lysozyme |
| $IC_{50}$ | Concentration leading to 50% inhibition |
| IFN$\alpha$ | Interferon alpha |
| Ig | Immunoglobulin |
| IL | Interleukin |
| InsR | Insulin receptor |
| LLOQ | lower limit of quantification |
| LPS | Lipopolysaccharide |
| m-IL-3 | murine IL-3 |
| MLR | Mixed lymphocyte reaction |
| mTOR | Mammalian target of rapamycin |
| n.d. | not determined |
| PBMC | Peripheral blood mononuclear cells |
| pDC | Plasmacytoid dendritic cell |
| PDK1 | 3-Phosphoinositide-dependent protein kinase 1 |
| PH | Pleckstrin Homology domain |
| PI | Phosphatidyl-inositol |
| PI3K | Phosphatidyl-inositol 3-kinase |
| PKB | Protein kinase B (also known as Akt) |
| rIL-4 | Rat interleukin-4 |
| RT | Room Temperature |
| SCF | Stem cell factor |
| SD | Standard deviation |
| SEM | Standard error of the mean |
| Ser473 | Serine position 473 |
| Th | T helper cell |
| TLR | Toll-like receptor |

**Human B cell activation in diluted whole blood.**

**Method.** For assessing effects on B cell activation upon surface B cell receptor stimulation, B lymphocytes in 90% human whole blood were stimulated by incubation with anti-IgM antibodies alone (aIgM) or in combination with IL-4 (algM/IL-4) in the presence of titrated amounts of compounds. Stimulations in whole blood closely reflect the physiological condition and take potential binding to plasma proteins into account. Early activation via the pathway proximal to the target PI3K was visualized as the inhibition of Akt phosphorylation.

For assessment of the *in vitro* effects of compound **A** on B cell surface activation markers upon stimulation, 180.5 μl heparinised whole blood was spiked with 9.5 μl of pre-diluted compound **A** in 96 well U-bottomed microtiter plates (Nunc) resulting in a 2-fold serial dilution with a concentration range from 50 to 0.008 μM. Control wells were pretreated with DMSO to obtain a final concentration of 0.5 % DMSO. Cultures were set up in duplicates, mixed well by agitation on a plate shaker (30 sec, speed 900), pipetting up and down and agitated on the plate shaker again. Cultures were incubated at 37°C, 5% $CO_2$ for 1 hr. Then 10 μl stimulus solution as described and mixed as above and cultures were incubated at 37°C, 5% $CO_2$ for a further 24 h.

*Flow cytometry analysis*

Following incubation, cell aggregates were broken up by addition of 15 μl/well of a 25 mM EDTA solution, pH 7.4, to the cultures. Samples were mixed thoroughly by agitation on a plate shaker (speed 900) for 15 min. Cells were stained by addition of 25 μl of a mixture of fluorescent labelled antibiodies, mixing on a plate shaker (30 sec, speed 900) and incubation for 30 min in the dark at room temperature. Samples were stained with anti-huCD3-APC-Cy7 (Becton Dickinson [BD] # 557832) to allow gating on T cells and with anti-huCD19-APC (BD # 555415) to allow gating on B cells in FACS analysis. In addition, samples were stained with combinations of the following antibodies as described in the Results section: anti-huCD69-PE-Cy7 (BD # 557745) and anti-huCD86-PE-Cy5 (BD # 555659).

After staining, samples were transferred to 96-deep well V-bottomed microtiter plates, (Corning # 396096) containing 2 ml/well of 1x BD Lysing Solution (BD # 349202). Plates were mixed by pipetting up and down and incubated for 10 min in the dark at room temperature. Plates were centrifuged at 450 x g for 5 min and after removal of the supernatant, 2 ml of CellWASH (BD # 349524) was added to each well. Plates were centrifuged at 450 x g for 5 min again, the supernatant removed and the cell pellet resuspended in 0.5 ml CellWASH.

Data was acquired on a BD LSR II flow cytometer using BD FACSDiva software (version 4.1.2). Lymphocytes were gated in the FSC/SSC dot plot according to size and granularity and further analyzed for expression of CD19, CD3, and activation markers. Data were calculated from dot blots as percentage of cells positively stained for activation markers within the CD19+ or CD3+ population.

*Statistical evaluation*

Signal-to-noise-ratios (S/N) were calculated by dividing the percentages of marker-positive B or T cells from activated blood samples by the percentage of marker-positive B or T cells from non activated samples.

The percentage inhibition of B or T cell activation after exposure to drug was calculated by the following formula:

$$\%\ \text{Inhibition} = 100\ \text{x}\ \frac{\text{stimulation without drug} - \text{stimulation with drug}}{\text{stimulation without drug} - \text{unstimulated}}$$

ORIGIN 7 software (OriginLab Corporation, Northampton, MA) was used for non-linear regression curve fitting. The drug concentration resulting in 50 % inhibition (IC50) were obtained by fitting the Hill equation to inhibition data.

For assessment of the in vitro effects of compound **A** on the intracellular pathway activation marker pAkt upon stimulation, 180 μl heparinized blood was spiked with 10 μl of pre-diluted compound **A** in 5 ml U-bottom tubes (BD, cat# 352063) resulting in a dilution with a concentration range from 16666 nM to 0.8 nM. Control samples were pretreated with DMSO to obtain a final concentration of 0.17 % DMSO. Samples were set up in duplicates, mixed well by agitation on a vortex (3 times 5 sec, speed 1800). Samples were incubated at 37°C in the water bath for 1.5 hrs (lids closed). Then, the stimulus in a volume of 10 μl was added, mixed (3 times 5 sec, speed 1800) and incubated for 20 min at 37°C in the water bath.

*Lysis, Fixation and Permeabilisation*

After incubation, 2 ml of pre-warmed (37°C in water bath) BD Phosflow Lyse/Fix buffer (BD, cat# 558049) was added per tube and shaken for 3 seconds on a vortexer and incubated for 20 min at 37°C in the water bath. Samples were centrifuged at 400g for 5 min. After centrifugation 2 ml of BD Phosflow Perm/wash buffer I (BD, cat# 557885) was added per tube and incubated at room temperature (RT) in the dark for 10 min. After centrifugation at 400 g for 5 min the pellets were washed with 2 ml of BD Phosflow Perm/wash buffer I and again centrifuged at 400 g for 5 min. Supernatants were discarded and the samples were stained as described below.

*Flow cytometry analysis*

For analysis of pAkt, processed human blood samples were stained with anti-hu CD20 (Alexa488-labeled anti-huCD20, BD cat# 558056) to allow gating on B cells in the cytometric analysis. In addition, samples were stained with Alexa647 conjugated anti-human phospho-Akt (Ser473; BD, cat# 560343).

Staining procedures were performed in BD Phosflow Perm/wash buffer I at RT for 30 min in the dark. After incubation, samples were washed with 2 ml of BD Phosflow Perm/wash buffer I and centrifuged at 400 g for 5 minutes and the pellets were resuspended in 300 μl BD Stain Buffer (BD, cat# 554656). Samples were kept on ice until data were acquired on an LSRII flow cytometer (BD Biosciences) using DIVA (version 6.1.2) software. Lymphocytes were gated in the FSC/SSC dot blot according to size and granularity and further analyzed for expression of CD20 and phosphorylation of Akt. Data were calculated from dot blots or histograms as percentage of cells positively stained for Akt-phosphorylation within the CD20+ population. Statistical ecvaluation was performed as described above for surface activation markers.

**Results.** (Table 1).

*Table 1 Inhibition of human B cell functions in whole blood*

| Matrix | Human 90% blood | | |
|---|---|---|---|
| Stimulus | aIgM/IL-4 | | aIgM |
| Readout | CD69 | CD86 | pAkt[e] |
| (A) $IC_{50}$ [μM] | 0.193[d] | 0.202[d] | 0.144 |

[d]Shown data are mean values ± SD of at least four independent experiments; [e]Shown data are mean values ± SD of at least two independent experiments

**Mouse B cell proliferation following BCR stimulation.**

**Method.** Murine B cells were stimulated via BCR by anti-IgM antibody in the presence of titrated amounts of compounds as described in (Julius et al 1984) and proliferation was assessed by incorporation of radioactive $^3$H-Thymidine.

**Results.** (Table 2).

**LPS-induced antibody production from mouse B cells in vitro.**

**Method.** The LPS-induced B cell functions were investigated according to a protocol adapted from Moon (Moon et al 1989) with minor modifications: Splenic B cell from Balb/c nu/nu mice were cultured with mIL-4, mIL-5 and LPS in the presence of titrated amounts of compound. The final concentrations were 0.5x$10^5$ splenocytes/well, 500 U/ml mIL-4, 500 U/ml mIL-5, and 50 μg/ml LPS. Supernatants were analyzed for antibody production by ELISA after 6 days of incubation.

**Results.** (Table 2).

**Antigen presentation and cytokine production of mouse BCR transgenic B cells. Method.** Purification of $CD19^+$ splenic B cells from MD4 HEL BcR Tg mice

MD4 HEL BcR transgenic B10.BR (MD4 B10.BR) mice were a kind gift from Prof. Jose Moreno (Research Unit on Autoimmune Diseases, Centro Medico Nacional Siglo XXI, Mexico).

The spleens were isolated from MD4 B10.BR and non-transgenic litter control B10.BR mice after sacrificed by exposure to excess amount of isoflurane. The isolated spleens were suspended in RPMI1640 (Invitrogen, #31870), and dissociated by GentleMACS Dissociator (Miltenyi Biotec), and filtrated with Cell Strainer (BD Falcon, 70 μm mesh, #352350). Single spleen cell suspension was further treated with lysing buffer (Sigma, #R7757) to remove erythrocytes, washed with $PBS^-$ twice, and re-suspended in the complete culture medium consisting of RPMI-1640 supplemented with 10% FBS, 100 U/ml penicillin, 100 μg/ml streptomycin, and 50 μM 2-mercaptoethanol (2-ME). Further splenic B cell purification was performed via depletion of non-B cells with magnetic cell sorting, AutoMACS (Miltenyi Biotec) according to the manufacture's instruction provided. In brief, $1 \times 10^7$ cells of spleen cells suspended in 40 μl of MACS buffer, PBS supplemented with 0.5% bovine serum albumin (BSA) and 2 mM EDTA were incubated with 10 μl of biotin-antibodies cocktail consisting of monoclonal antibodies against CD43 (Ly48, rat $IgG_{2a}$), CD4 (L3T4, rat $IgG_{2b}$), and Ter-119 (rat $IgG_{2b}$) for 15 minutes on ice. After treating with biotin-antibodies cocktail, splenocytes were further incubated

with 30 μl of anti-biotin antibodies for 15 minutes on ice, re-suspended in 1 ml of MACS buffer and applied to purify B cells via AutoMACS. In AutoMACS purification "Deplete" program was chosen, and the negative fraction from outlet port neg1 was collected as the B cell-rich fraction. The purity was determined via the proportion of $CD19^+$ cells in the separated fraction in FACS analysis, and was more than 95%.

*Cell staining and FACS analysis*

Purified B cells were re-suspended in 50 μl of ice-cold FACS buffer (PBS supplemented with 0.1% azide and 0.1% BSA). Cell suspensions in FACS buffer were treated with 1 μl of Fc block (rat Anti-Mouse CD16/CD32 antibody, BD Pharmingen, #553142) for 10 minutes on ice. After treatment with Fc block, cells were stained for 30 minutes on ice with anti-CD19 PerCp to identify B cell population, and further with anti-$HEL_{48-61}$ peptide/MHC class II I-Ak (Aw3.18.14) antibodies followed by the anti-mouse $IgG_1$ PE antibody to measure cell activation as well as antigen presenting activity. Stained cell samples were washed with 5 ml of ice-cold FACS buffer twice, and analyzed by FACS Calibur (BD Bioscience).

The Aw3.18.14 antibody (Dadaglio G et al. 1997) was purified from hybridoma culture (ATCC, #CRL2826) with Amicon centrifuge 30kd filter (Milliore, #LSK2ABA20). All other antibodies were purchased from BD Bioscience.

FACS Data was analyzed, and their mean fluorescence intensity (MFI) were calculated with FlowJo software (Tree Star Inc). The $IC_{50}$ values were calculated with GraphPad PRISM ver 6.0 software (GraphPad Software Inc).

*The measurement of antigen-loading via MHC II*

One million of splenocytes or purified B cells from MD4 B10.BR mice was suspended in 500 μl of complete culture medium, and seeded in 24 wells plate. The cells were pre-treated with compound **A** for 30 mins, and further cultured with certain concentrations of HEL protein at 37 °C, 5 % $CO_2$ over night. After the culture over night, the cells were harvested, and applied to FACS analysis to measure expression level of HEL peptide/MHC II complex on $CD19^+$ cells as described above. DMSO was kept at the concentration less than 0.1%.

*Measurement of proinflammatory cytokine release*

One million of B cells purified from MD4 B10.BR as written in the section 2.1 was suspended in 200 μl of complete culture medium, and stimulated with 100 μM of HEL proteins with soluble CD40 ligand at 37 °C, 5 % $CO_2$ for 48 hours. Compound A was added in the culture 30 minutes before the stimulation. After the stimulation with HEL protein, culture supernatants were harvested, and applied to measure IL-6 and TNFα by ELISA according to manufature's instruction provided (R&D systems). The data are represented as the mean of concentration (pg/ml) from triplicate samples, and $IC_{50}$ values are calculated as described above. The concentration of DMSO was kept at the concentration less than 0.1%.

**Results.** (Table 2).

*Table 2 Inhibition of murine B cell functions*

| Matrix | Mouse spleen | | Mouse BCR tg B cell | | |
|---|---|---|---|---|---|
| Stimulus | Anti-IgM | LPS/ IL-4 | Hen Egg Lysozyme (HEL) | | |
| Readout | Proliferation | IgM | function | IL-6 | TNFα |
| (**A**) $IC_{50}$ [μM] | 0.008[a] | 0.234[a] | 0.395[b] | 0.022[a] | 0.028[a] |

[a]Shown data are mean of at least two independent experiments; [b]Shown data are mean of at least four independent experiments

## Chemokine production of human peripheral blood mononuclear cells (PBMC)

**Method.**

*Isolation of PBMC*

For evaluating the effect of compound **A** on the production of chemokines relevant for the formation of germinal centers, PBMC were isolated from Buffy Coat (collected through Inter-Regionale Blutspende of the Swiss Red Cross) by standard Ficoll gradient centrifugation using Ficoll-Paque plus (GE healthcare # 17-1440-03) in Leucosep tubes

(Greiner Bio-one # 227289). Cells were washed two times with PBS and then resuspended at 2.2 x $10^6$/mL in RPMI 1640 medium supplemented with 10 % FBS, Gentamycine (50 ug/ml), Insulin-Transferrin-Selenium and β-Mercaptoethanol (50 uM).

*Stimulation of PBMCs*

PBMC were dispensed to 48-well plates (Costar #3548) and incubated with pre-diluted compound **A** resulting in a dilution with a final concentration of 0.3, 1 or 3 uM. Control samples were pretreated with DMSO to obtain a final concentration of 0.03 % DMSO. Samples were mixed thoroughly and incubated in a humified incubator at 37 °C for 1 h. Cells were thereafter stimulated by the addition of the TLR9 agonist ODN M362 (Invivogen, #tlrl-m362) at a final concentration of 30 ug/ml or anti-IgM-Dextran (Finabiosolution #0004) at a final concentration of 2 ug/ml. Control samples were left unstimulated. Samples were mixed thoroughly and incubated in a humified incubator at 37 °C for 24 h.

Cvtokine/chemokine determination

After the stimulation, culture supernatants were harvested and chemokines quantified by Bio-Plex Multiplex immunoassay (CXCL13; Bio Rad #171 BK12MR2) or MSD V-Plex assay (IP-10; Meso Scale Diagnostics) or according to the manufaturer's instructions.

**Results.** (Table 3 and 4).

*Table 3 Inhibition of TLR9 ligand-induced CXCL 13 production*

| stimulus | no | ODN | ODN | ODN | ODN |
|---|---|---|---|---|---|
| Compound **A** (nM) | no | no | 3000 | 1000 | 300 |
| Donor 1 | 1[a] | 61 | 41 | 42 | 51 |
| Donor 2 | 1 | 19 | 4 | 6 | 8 |
| Donor 3 | 0 | 14 | 5 | 7 | 8 |
| Donor 4 | 1 | 9 | 3 | 3 | 3 |
| Donor 5 | 1 | 63 | 29 | 42 | 39 |
| Donor 6 | 4 | 202 | 86 | 84 | 113 |
| mean | 1 | 61 | 28 | 31 | 37 |
| SD | 1 | 73 | 33 | 32 | 42 |
| % inhibition | | | 55 | 51 | 40 |

[a] Quantity of measured CXCL13 in culture supernatant shown as pg/ml

*Table 4 Inhibition of anti-IgM-Dextran ligand-induced IP-10 production*

| stimulus | no | anti-IgM-Dextran | anti-IgM-Dextran | anti-IgM-Dextran | anti-IgM-Dextran |
|---|---|---|---|---|---|
| Compound **A** (nM) | no | no | 3000 | 1000 | 300 |
| Donor 1 | 117 | 3026 | 715 | 1624 | 2764 |
| Donor 2 | 48 | 106 | 93 | 71 | 79 |
| Donor 3 | 15 | 77 | 27 | 29 | 32 |
| Donor 4 | 17 | 35 | 34 | 42 | 37 |
| Donor 5 | 88 | 1460 | 899 | 1010 | 1233 |
| Donor 6 | 442 | 58 | 59 | 52 | 52 |
| mean | 121 | 794 | 305 | 471 | 699 |
| SD | 162 | 1227 | 394 | 683 | 1117 |
| % inhibition | | | 62 | 41 | 12 |

[a]Quantity of measured IP-10 in culture supernatant shown as pg/ml

## CXCL13-induced B cell migration

**Method.**

*Isolation of B cells from Buffy Coat*

PBMC were isolated from Buffy Coat as described above. Cells were washed two times with PBS and then resuspended at 5 x $10^7$/mL in PBS containing 2% FBS and 1 mM EDTA. Further B cell purification was performed via depletion of non-B cells with an EasySep™ Human B Cell Enrichment Kit (STEMCELL Technologies # 19054) according to the manufacture's instruction. In brief, 4 x $10^8$ PBMCs in 8 ml isolation buffer were incubated with 400 ul of enrichment cocktail in a 14 mL round-bottom tube for 10 minutes at room temperature. PBMC were further incubated with 600 ul of magnetic particles for 5 minutes at room. For B cell isolation, the tube was placed into the magnet for 5 min and enriched B cells were collected in a fresh 14 ml tube. Cells were then washed and resuspended at 5 x $10^6$/mL in RPMI 1640 medium supplemented with 10 % FBS, Gentamycine (50 ug/ml), Insulin-Transferrin-Selenium and β-Mercaptoethanol (50 uM).

Migration assay in 96-well transwell plate

Isolated B cells were dispensed to 5 ml round-bottom tube (Costar # 352054) and incubated with pre-diluted compound **A** resulting in a dilution with a final concentration of 10, 1, 0.1 or 0.01 uM. Control samples were pretreated with DMSO to obtain a final concentration of 0.1 % DMSO. Samples were mixed thoroughly and incubated in a water bath at 37 °C for 30 minutes. Some 235 ul of CXCL13 (R&D Systems # 801-CX) in a dilution with a final concentration of 100 nM was added to the wells of the 96-well transwell receiver plate (Costar # 3387). Control wells were filled with 235 ul medium. The 5 um permeable support insert was added to the receiver plate and filled with 80 ul of pre-incubated B cells. Transwell plate was incubated in a humified incubator at 37 °C for 3 h. Permeable support insert was removed and cell number in receiver plate was assessed by flow cytometry. **Results.** (Table 5).

*Table 5 Inhibition of CXCL 13-induced B cell migration*

| stimulation | no | CXCL13 | CXCL13 | CXCL13 | CXCL13 | CXCL13 |
|---|---|---|---|---|---|---|
| compound **A** (nM) | no | no | 10000 | 1000 | 100 | 10 |
| cell count[a] | **737** | **6981** | **2043** | **3743** | **4556** | **6165** |
| SD | 42 | 478 | 353 | 594 | 317 | 953 |
| % inhibition | **100** | **0** | **79** | **52** | **39** | **13** |
| StD | 1 | 8 | 6 | 10 | 5 | 15 |

[a] Mean value of 4 wells

## Time-dependent inhibition of Akt-phosphorylation *ex vivo* by a single oral dose of test compound in rats

**Method.**

*Animals*

All experiments were performed with adult male Lewis rats (LEW/Han/Hsd, Charles River, Germany and LEW/Orl@Rj, Janvier, France), weighing 220-280 g.

*Maintenance Conditions*

Animals were housed under conventional hygienic conditions and fed a standard diet and drinking water ad libitum. They were allowed unrestricted access to food and water before and during the experiment.

*Reagents*

High molecular weight sodium heparin (B.Braun, Melsungen, Germany; 5000 I.U./ml) was used as anticoagulant. Goat anti-rat IgM antibody was obtained from Serotec, Düsseldorf Germany (cat# 302001). BD Phosflow Lyse/Fix

buffer I was obtained from BD biosciences (cat# 558049). Recombinant rat IL-4 (BD, cat# 555107) was stored in aliquots at -80°C.

*Drugs and drug application in vivo*

The suspension for administration was freshly prepared and stored in the dark at room temperature. 14.8 mg of compound **A** was suspended in 5.92 ml CMC 0.5% with 0.5% Tween80. The milky, homogeneous suspensions were then applied to the animal. The test substances were administered p.o. at in a volume of 4 ml/kg body weight resulting in an oral dose of 10 mg/kg.

*Blood collection*

Animals were anaesthetized with isoflurane using a Fluvac airflow system. Whole blood was collected sublingually pre-dose and at 1, 2, 4, 6, 8, 10, 12 and 24 hours post-dose. For pharmacodynamic analysis 100μl rat blood was collected per time point in Eppendorf tubes with 30 IU sodium heparin (B.Braun, Melsungen, Germany; 5000 I.U./ml). For pharmacokinetic analysis 150 μl rat blood was collected per time point in EDTA coated Eppendorf (Milian cat# TOM-14) tubes.

*Pharmacokinetic analysis*

For pharmacokinetic analysis 150 μl of each whole blood sample was stored at -80°C prior analysis. After addition of 20 μL internal standard (conc = 400 ng/mL) to an 80 μL aliquot of each whole blood sample, cells and proteins were removed by centrifugation following precipitation with 400 μL acetonitrile. The organic upper layer was then evaporated to dryness. The residues were dissolved in 50 % acetonitrile containing 0.2 % formic acid, diluted with 0.2% formic acid, centrifuged and then stored at 10 °C prior to analysis. For calibration, 8 blank whole blood samples were spiked with amounts of compound from 1 to 5000 ng/ml. Quality and recovery control samples were set to 100 ng/ml.

For analysis a 10 μL aliquot of each sample extract was injected into a LC-MS-MS system. Compounds were resolved on a Reprosil-pur C18 reversed phase column applying a linear gradient from 5 mM ammonium formate containing 0.2 % formic acid to acetonitrile, containing 5 % methanol, within 5 minutes. For detection, mass spectrometry in MRM with the mass transition 451.2 m/z → 247.0 m/z was used. After ionization of the column effluent in an AP electrospray source, compound **A** and internal standard were detected as their [MH]+ product ions.

Compound **A** concentrations were calculated by XKalibur® and Excel®, based on the extracted peak area ratio, obtained from the relative intensity of the MS/MS signal. The LOQ was determined to be 1 ng/ml and the accuracy of the calibration between 1 ng/ml and 1000 ng/ml was better than 5%. The precision of the QC samples (n = 5 at 100 ng/ml) was better than 4.7% RSD and the recovery was 102.6%.

*Whole blood stimulation in vitro*

For ex vivo analysis of blood from drug-treated animals, 90 ml heparinized blood was mixed in 5ml U-bottom tubes (BD, cat# 352063) with 100 ml of RPMI medium (Gibco, cat# 31870) immediately after blood collection, and activated with 10 μl of anti-rat IgM/rIL-4 at a final concentration of anti-rat IgM of 50 mg/ml and rIL-4 of 10 ng/ml. Control samples were left unstimulated. Samples were mixed thoroughly and incubated for 10 minutes at 37°C in the water bath.

*Lysis, Fixation and Permeabilisation*

After incubation, 2 ml of pre-warmed (37°C in water bath) BD Phosflow Lyse/Fix buffer was added per tube. Samples were mixed thoroughly and incubated for 20 minutes at 37°C in the water bath. Then samples were centrifuged at 400g for 5 minutes. After centrifugation 2 ml of BD Phosflow Perm/wash buffer was added per tube. Samples were mixed thoroughly and incubated at room temperature (RT) in the dark for 10 minutes. Thereafter, the samples were snap-frozen on dry ice and then stored at -80 °C until FACS staining.

*Flow cytometry analysis*

For thawing, all samples were incubated for 10 minutes in the water bath at 25°C. After centrifugation at 400g for 5 minutes the pellets were washed with 2 ml of BD Phosflow Perm/wash buffer and again centrifuged at 400g for 5 minutes. Supernatants were removed. For analysis of pAkt, samples were stained with PE-labeled anti-rat IgM (BD, cat# 553888) to allow gating on B cells in FACS analysis. In addition, samples were stained with Alexa647 conjugated anti-phospho-Akt (Ser473; BD, cat# 560343). Staining procedures were performed in a total volume of 100 μl BD

Phosflow Perm/wash buffer at RT for 30 minutes in the dark. After incubation, samples were washed with 2 ml of BD Phosflow Perm/wash buffer and centrifuged at 400g for 5 minutes and the pellets were resuspended in 300μl BD Stain Buffer (BD, cat# 554656). Samples were kept on ice until data were acquired on an LSRII flow cytometer (BD Biosciences) using DIVA software (version 6.1.2). Lymphocytes were gated in the FSC/SSC dot blot according to size and granularity and further analyzed for expression of IgM and phosphorylation of Akt. Data were calculated from dot blots or histograms as percentage of cells positively stained for Akt-phosphorylation within the IgM+ population.

*Statistical evaluation*

The effects of the drugs ex vivo were expressed as inhibition of Akt-phosphorylation measured by flow cytometry. The percentage inhibition of Akt-phosphorylation was calculated by the following formula:

$$\% \text{ Inhibition} = 100 \text{ x } \frac{\text{stimulation without drug} - \text{stimulation with drug}}{\text{stimulation without drug} - \text{unstimulated}}$$

**Results.** (Table 6).

*Table 6 Time-dependent inhibition of Akt-phosphorylation in rat*

| | **% inhibition of pAkt in IgM+ B cells** | | | | | |
|---|---|---|---|---|---|---|
| **time [h]** | **rat 1** | **rat 2** | **rat 3** | **rat 4** | **Average** | **SD.** |
| | | | | | | |
| 0 | -2.0 | 15.7 | 6.9 | -20.6 | **0.0** | **15.5** |
| 1 | 100.0 | 98.2 | 95.6 | 99.1 | **98.2** | **1.9** |
| 2 | 100.9 | 97.3 | 101.8 | 98.2 | **99.6** | **2.1** |
| 4 | 93.8 | 100.0 | 105.3 | 81.4 | **95.1** | **10.3** |
| 6 | 76.1 | 99.1 | 94.7 | 83.1 | **88.2** | **10.6** |
| 8 | 83.1 | 83.1 | 85.8 | 65.4 | **79.4** | **9.4** |
| 10 | 69.0 | 74.3 | 76.9 | 62.7 | **70.7** | **6.3** |
| 12 | 74.3 | 71.6 | 73.4 | 69.0 | **72.1** | **2.3** |
| 24 | 30.8 | 40.6 | 37.0 | 12.2 | **30.2** | **12.6** |

Group of four Lewis rats were treated with a single oral dose of 10 mg/kg of compound **A.** At indicated time points, 50% rat whole blood was stimulated with anti-rat IgM/rIL-4 and Akt-phosphorylation was determined as described in the Method section. Data show individual and mean values of four animals with SD.

**Pharmacokinetic analysis of compound A in human plasma**

**Method.** A whole blood sample was obtained by either direct venipuncture or an indwelling cannula inserted in a forearm vein. Blood samples were collected into ethylenediaminetetraacetic acid tri potassium (K3 EDTA) containing tubes and centrifuged within 60 minutes at 3- 5°C to separate plasma. The plasma obtained was frozen and stored at -20°C until drug concentration measurement.

Plasma concentrations of compound **A** were quantified using a validated electrospray ionization liquid chromatography-tandem mass spectrometry method (HPLC-MS/MS) in positive ion mode. Briefly, 40 μl of plasma samples were transferred to an Impact protein precipitation plate placed on a 96 well plate. One hundred and fifty microliters of internal standard [13CD3] compound **A** diluted to 4 ng/mL prepared in 75% acetonitrile in water were added into the 96 well plate before vortex-mixing. For control blank samples a volume of one hundred and fifty microliters of 75% acetonitrile in water not containing internal standard was added in replacement. The plate sealed with a film was shaken for 10 minutes, centrifuged (10 minutes, 2250 g, at 4°C). A volume of 4 μL of the sample extract was injected into the LC/MS/MS system (API4000, Applied Biosystems).

The chromatographic separation of compound **A** was conducted using an Ascentis 2.7 μm C18, 50 × 2.1 mm (Sigma-Aldrich) at 40°C and a flow rate of 1.00 ml/min. The mobile phase consisted with A: 0.1% formic acid in water

and B: 0.1% formic acid in acetonitrile. The initial condition with 10% of mobile phase B was maintained for the first 0.3 min, the composition of mobile phase B was increased linearly to 50% in the next 0.3 min, jumped to 95% in 1.0 min and then back to 10%. Retention time for compound **A** and its internal standard was ca. 1.4 min. The selected mass transitions were respectively 451.2→247.1, 174.1 and 455.3→251.2, 174.2, 124.2 for compound **A** and [13CD3] compound **A.**

Calibration curves were constructed using peak area ratios (compound **A** versus [13CD3] compound **A**) of the calibration standards by applying a weighted (1/concentration squared) quadratic least squares regression algorithm.

The compound **A** concentrations in clinical samples were back-calculated from their peak area ratios against the calibration curve (Analyst software). The method was successfully validated over the range of 3 ng/mL to 1000 ng/mL with a LLOQ of 3 ng/mL. The dynamic range was covered by 7 calibration standards. The method fulfilled the given criteria for acceptance regarding linearity (calibration standards deviation ≤15% (≤ 20% at the LLOQ) from the calibration curve), inter-day and intra-day accuracy and precision (mean bias ≤ 15% (≤ 20% at the LLOQ) of the nominal value; precision ≤ 15% (≤ 20% at the LLOQ)) as well as carry-over response.

**Results.** (Figure 1).

**Measurement of pAkt in B cells in stimulated/non-stimulated whole blood clinical samples**

**Method.**

*Blood collection and cell stimulation*

For assessment of the *in vivo* effects of compound **A** on human B cell activation measured as Akt phosphorylation vlinical samples were prepared from whole blood using a Na-Heparin monovette system.

Whole blood was then incubated with either anti-IgM/IL-4 (7.5 μg/ml of anti-IgM [Southern Biotech] and 7.5 ng/ml of IL-4 [R&D]) or with PBS (unstimulated sample). Incubation was performed using 95 μl of blood (in duplicates) in plate format within 1 h after blood collection. Cultures were mixed well by gently pipetting up and down and incubated at 37°C (in incubator, 5% $CO_2$). After 30 min whole blood was lysed and fixed with pre-warmed (37°C) BD PhosFlow-Lyse/Fix buffer (1600 μl) for 20 minutes at 37°C (water bath) in the dark. The lysed and fixed blood samples were immediately frozen and stored at -80°C until further processing.

*Staining for B cell activation marker*

Clinical samples were processed within one week after blood collection. After thawing was completed (at 37°C water-bath), samples were centrifuged at 502g (1580 rpm) for 5 min at room temperature. Then pelleted cells were washed with 500 μL ice cold BD Phosflow Perm II buffer, mixed well and kept on ice for 30-35 minutes. Following incubation, 1200 μl FACS buffer was added and cells were centrifuged for 10 min at 650 g (1800 rpm) at room temperature. Supernatants were discarded and the samples were washed once again as described above.Staining procedure consisted of both direct staining (with anti-human CD20 Per-CP-Cy5.5-labeled [BD cat# 558021] and anti-human total Akt Alexa Fluor® 488 Conjugated [Cell Signaling cat# 2917S]), and indirect staining for pAkt (Ser473) (Cell Signaling cat# 4058L). Firstly cells were incubated with primary antibodies (50 μL total volume) for 30-35 min at room temperature in the dark followed by washing with FACS buffer (1200 μL), and centrifugation at 650 g (1800 rpm) for 10 min at RT. Pelleted cells were further incubated with secondary Ab (50 μL total volume) for 15-17 min at RT in the dark followed by washing with FACS buffer (1200 μL), and centrifugation at 650 g (1800 rpm) for 10 min at RT. After repetition of washing step (FACS buffer 1200 μL, centrifugation at 650 g for 10 min at RT), cells were resuspended in 300 μL 1% PFA (in PBS) and transferred to 5 mL polystyrene tubes.

*Data acquisition and analysis*

Samples were analyzed on the same day. Data acquisition was performed on a FACS Canto II using the DIVA software at high flow-rate. Gating was be as follows: Firstly doublets were excluded using a FSC-A vs. FSC-H dot plot. Single cells were then displayed on a FSC-A vs. SSC-A dot plot to create a gate on the leukocytes. A CD20 vs. SSC-A dot plot was created and leukocytes were displayed. Based on CD20-positive B cells histogram plot for pAkt was created. Unstimulated samples were used to set the interval gate for positive and negative pAkt populations. Data was calculated as percent inhibition of pAkt relative to the baseline value.

**Results.** (Figure 1).

Compound **A** shows a dose-, concentration- and time-dependent inhibition of the PI3K/Akt pathway. A pathway inhibition of greater than 80% over 12 hours is achieved after a single administration of 80 mg compound **A.** Modeling of the dose-response relationship for compound **A** would suggest that a sustained pathway inhibition over 24 hours

would be ensured by a BID dosing regimen.

## Claims

1. 1-{(S)-3-[6-(6-Methoxy-5-trifluoromethyl-pyridin-3-yl)-5,6,7,8-tetrahydro-pyrido[4,3-d]pyrimidin-4-ylamino]-pyrrolidin-1-yl}-propan-1-one or pharmaceutically acceptable salts thereof for use in the treatment of primary Sjögren's Syndrome.

2. A compound for use according to claim 1, which is the phosphate salt of 1-{(S)-3-[6-(6-methoxy-5-trifluoromethyl-pyridin-3-yl)-5,6,7,8-tetrahydro-pyrido[4,3-d]pyrimidin-4-ylamino]-pyrrolidin-1-yl}-propan-1-one.

3. A pharmaceutical composition comprising a therapeutically effective amount of a compound for use according to claim 1 or 2 and one or more pharmaceutically acceptable carriers.

4. A combination comprising a therapeutically effective amount of a compound for use according to claim 1 or 2 and one or more therapeutically active agents.

5. A compound for use according to claim 1 or claim 2 wherein the unit dosage is 10-100 mg of active ingredient for a human subject of about 40-200 kg.

6. A compound for use according to claim 1 or claim 2 wherein the unit dosage is 70 mg of active ingredient for a human subject of about 50-70 kg.

7. A compound for use according to claim 5 or claim 6 wherein the administration is b.i.d..

## Patentansprüche

1. 1-{(S)-3-[6-(6-Methoxy-5-trifluormethyl-pyridin-3-yl)-5,6,7,8-tetrahydro-pyrido[4,3-d]pyrimidin-4-ylamino]-pyrrolidin-1-yl)-propan-1-on oder pharmazeutisch akzeptable Salze davon zur Verwendung bei der Behandlung des primären Sjögren-Syndroms.

2. Verbindung zur Verwendung nach Anspruch 1, die das Phosphatsalz von 1-{(S)-3-[6-(6-Methoxy-5-trifluormethyl-pyridin-3-yl)-5,6,7,8-tetrahydro-pyrido[4,3-d]pyrimidin-4-ylamino]-pyrrolidin-1-yl)-propan-1-on ist.

3. Pharmazeutische Zusammensetzung, umfassend eine therapeutisch wirksame Menge einer Verbindung zur Verwendung nach Anspruch 1 oder 2 und ein oder mehrere pharmazeutische akzeptable Trägerstoffe.

4. Kombination, umfassend eine therapeutisch wirksame Menge einer Verbindung zur Verwendung nach Anspruch 1 oder 2 und einen oder mehrere therapeutisch wirksame Stoffe.

5. Verbindung zur Verwendung nach Anspruch 1 oder Anspruch 2, wobei die Einheitsdosis 10-100 mg Wirkstoff für einen Menschen von etwa 40-200 kg beträgt.

6. Verbindung zur Verwendung nach Anspruch 1 oder Anspruch 2, wobei die Einheitsdosis 70 mg Wirkstoff für einen Menschen von etwa 50-70 kg beträgt.

7. Verbindung zur Verwendung nach Anspruch 5 oder Anspruch 6, wobei die Verabreichung b.i.d. erfolgt.

## Revendications

1. 1-{(S)-3-[6-(6-méthoxy-5-trifluorométhyl-pyridin-3-yl)-5,6,7,8-tétrahydropyrido[4,3-d]pyrimidin-4-ylamino]-pyrrolidin-1-yl}-propan-1-one ou sels pharmaceutiquement acceptables de celui-ci pour une utilisation dans le traitement du syndrome de Sjögren primaire.

2. Composé destiné à être utilisé selon la revendication 1, qui est le sel de phosphate de 1-{(S)-3-[6-(6-méthoxy-5-trifluorométhyl-pyridin-3-yl)-5,6,7,8-tétrahydro-pyrido[4,3-d]pyrimidin-4-ylamino]-pyrrolidin-1-yl}-propan-1-one.

**3.** Composition pharmaceutique comprenant une quantité thérapeutiquement efficace d'un composé destiné à être utilisé selon la revendication 1 ou 2 et un ou plusieurs excipients pharmaceutiquement acceptables.

**4.** Combinaison comprenant une quantité thérapeutiquement efficace d'un composé destiné à être utilisé selon la revendication 1 ou 2 et d'un ou plusieurs agents thérapeutiques actifs.

**5.** Composé destiné à être utilisé selon la revendication 1 ou 2, dans lequel la dose unitaire est de 10 à 100 mg d'ingrédient actif pour un sujet humain d'environ 40 à 200 kg.

**6.** Composé destiné à être utilisé selon la revendication 1 ou 2, dans lequel la dose unitaire est de 70 mg d'ingrédient actif pour un sujet humain d'environ 50 à 70 kg.

**7.** Composé destiné à être utilisé selon la revendication 5 ou 6, dans lequel l'administration est biquotidienne.

Figure 1

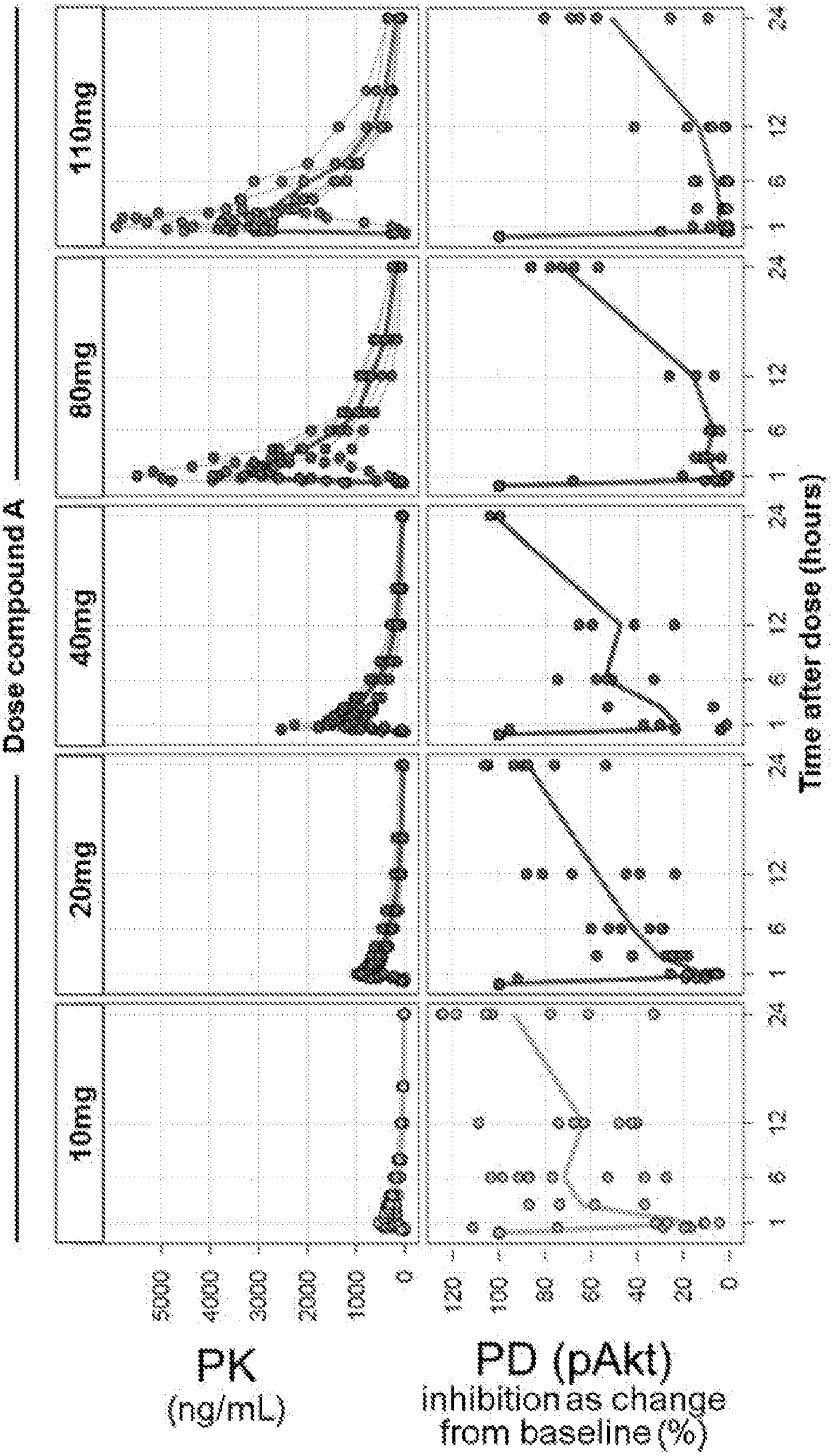
Dose compound A
10mg
20mg
40mg
80mg
110mg
PK
(ng/mL)
PD (pAkt)
inhibition as change
from baseline (%)
Time after dose (hours)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012004299 A
- WO 2012065950 A
- WO 2010007082 A


### Non-patent literature cited in the description


- **HANSEN A et al.** *Arthritis Research & Therapy,* 2007, vol. 9, 218
- **LEE, Y. J. et al.** *Rheumatology,* 2010, vol. 49 (9), 1747-1752
- **KRAMER JM et al.** *J Leukoc Biol.,* 2013, vol. 94 (5), 1079-1089
- **NISHIKAWA A et al.** *Arthritis Research & Therapy,* 2016, vol. 18, 106